# EUROPÄISCHE PATENTANMELDUNG

(11) **EP 3 323 433 A1**
(43) Veröffentlichungstag der Anmeldung: **23.05.2018**
(21) Anmeldenummer: 17001563.0
(22) Anmeldetag: 20.09.2017
(51) Int. Cl.: A61L 2/26, C02F 1/32, G01N 21/03, G01N 21/33, G01N 21/85, G01N 21/94, A61L 2/10, G01N 21/15

(54) **ARBEITSVERFAHREN UND VORRICHTUNG ZUR ÜBERWACHUNG EINER UV-DESINFEKTIONSANLAGE**

(30) Priorität: 17.11.2016 DE 102016122075
(71) Anmelder: Hytecon AG, 6006 Luzern (CH)
(72) Erfinder: Xu, Hongbin, 22149 Hamburg (DE); Kolch, Andreas, 32052 Herford (DE); Paulsmeyer, Dirk, 32423 Minden (DE)
(74) Vertreter: Rolf, Gudrun

(57) **Zusammenfassung**

Es werden ein Arbeitsverfahren und eine Vorrichtung einer UV-Desinfektionsanlage mit einer wasserdurchfluteten Reaktorkammer (1) und mindestens einer darin einstrahlen könnend angeordneten UV-Strahlungsquelle sowie mit einem UV-Sensor (3) und eine Auswerteeinheit für das Sensorsignal zur Verfügung gestellt, die mit einem geringen technischen Bauaufwand eine hohe Reinigungsleistung erzielen, die exakt für das gesamte bestrahlte Durchflussvolumen nachgewiesen werden kann, was dadurch erzielt wird, dass die Strahlungsquelle aus UV-LEDs besteht, die mindestens auf einer Seite der Reaktorkammer (1) angeordnet sind und dass der UV-Sensor (3) auf der gleichen Seite zwischen den UV-LEDs (2) angeordnet ist und dass mindestens die dieser Seite gegenüberliegende Innenfläche der Reaktorkammer (1) als Reflektionsfläche (4) für das UV-Licht ausgebildet ist.

## Beschreibung

Die Erfindung betrifft ein Arbeitsverfahren und eine Vorrichtung zur Überwachung einer UV-Desinfektionsanlage mit einer wasserdurchfluteten Reaktorkammer und mindestens einer darin einstrahlend könnend angeordneten UV-Strahlungsquelle sowie mit einem UV-Sensor und einer Auswerteeinheit für das Sensorsignal.

Eine solche Vorrichtung ist bereits bekannt, US 2003/0170151 A1, bei der sich eine Vielzahl von flächig angeordneten LEDs in den Durchfluss einer Flüssigkeit erstrecken, wobei in einigen Bereichen des Gehäuses Sensoren angeordnet sind, die die aktuellen Zustandswerte jeweils in dem entsprechenden Bereich detektieren und an eine Auswerteeinheit weitergeben.

Nachteilig an diesem vorbekannten System ist, dass nur ein geringer Teil des Volumenstromes einer Flüssigkeit detektiert werden kann, sodass über die Länge der Vorrichtung mehrere Sensoren angeordnet werden müssen, um ein aussagekräftiges Ergebnis zu erhalten. Bekannterweise wird jedoch eine Messung nur an einer "repräsentativen" Stelle durchgeführt, sodass keinesfalls eine vollständig korrekte Aussage über die tatsächlich erzielte Reinigungsleistung getroffen werden kann.

Aufgabe der Erfindung ist es, ein Arbeitsverfahren und eine Vorrichtung einer UV-Desinfektionsanlage zur Verfügung zu stellen, die mit einem geringen technischen Bauaufwand eine hohe Reinigungsleistung erzielen, die exakt für das gesamte bestrahlte Durchflussvolumen nachgewiesen werden kann.

Die Lösung dieser Aufgabe erfolgt in Verbindung mit den Merkmalen der Oberbegriffe der Ansprüche 1 und 7 erfindungsgemäß aus deren kennzeichnenden Teilen.

Dadurch, dass die Vorrichtung zur Überwachung einer UV-Desinfektionsanlage aus einer wasserdurchfluteten Reaktorkammer und mindestens einer darin einstrahlend könnend angeordneten UV-Strahlungsquelle, einem UV-Sensor und einer Auswerteeinheit für das Signal ausgestattet ist, bei der die Strahlungsquelle aus UV-LEDs besteht, die auf einer Seite der Reaktorkammer angeordnet sind und der UV-Sensor auf der gleichen Seite zwischen den UV-LEDs angeordnet ist und mindestens die dieser Seite gegenüberliegende Innenseite der Reaktorkammer als Reflektionsfläche für das UV-Licht ausgebildet ist, wird erreicht, dass die UV-Strahlung nach dem Einstrahlen in die Reaktorkammer bis zum Auftreffen auf den UV-Sensor die Reaktorkammer mindestens zweimal durchquert hat, da die UV-Strahlung nach dem Erreichen des Bodens der Reaktorkammer von dort bis zum Sensor zurückreflektiert wird, wodurch der Messbereich doppelt so lang ist wie das Gehäuse der Reaktorkammer tief und das Sensorsignal qualitativ verbessert ist.

Mit einem solchen UV-Sensor lassen sich etwa solche Parameter erfassen, wie die Leistung der UV-LEDs bzw. ggf. ein Nachlassen der selben, bedingt durch alterungsbedingte Prozesse oder eine Schwächung der UV-Strahlung durch Belagsbildung an der wasserzugewandten Seite oder aber durch im Wasser gelöste partikuläre Substanzen.

Vorteilhafte Ausgestaltungen des Gegenstandes der Erfindung ergeben sich mit und in Kombination aus den nachfolgenden Unteransprüchen.

Die neben dem UV-Sensor angeordneten UV-LEDs sind bevorzugterweise auf einem Trägermodul angeordnet und lassen sich in Abhängigkeit der erforderlichen Reinigungs- und Desinfektionsleistung einfach in ihrer Anzahl und Leistungsfähigkeit anpassen, wodurch mit der Vorrichtung **ein** großes Leistungsspektrum abgedeckt oder durch eine wahlfreie Auswahl an Trägermodulen des Weiteren mit unterschiedlichen Wellenlängen der UV-LEDs gearbeitet werden kann.

Entsprechend einer besonders bevorzugten Ausführungsform der Erfindung befindet sich das oder die Trägermodule außerhalb der wasserdurchfluteten Reaktorkammer, wobei die UV-LEDs unmittelbar auf einem Quarzfenster aufliegen, dessen den LEDs abgewandte Seite eine Reaktorinnenwand und den oberen Abschluss der Reaktorkammer bildet, sodass die UV-LEDs nicht mit der zu desinfizierenden Flüssigkeit in Kontakt kommen, aber durch das Quarzfenster keine Dämpfung ihrer Leistungsfähigkeit erfahren. Da die LEDs des Weiteren im Betrieb auf der dem Wasser zugewandten Seite praktisch kalt bleiben, wird auch das Quarzfenster nicht erwärmt, sodass keine Belagsbildung daran befürchtet werden muss, wie dies oftmals bei Reaktoren der Fall ist, die mit Gasentladungslampen als UV-Lichtquellen arbeiten.

Ein besonderer Vorteil der vorliegenden Vorrichtung besteht darin, dass die UV-LEDs so platziert und ausgerichtet sind, dass in der Reaktorkammer ein gleichmäßiges homogenes UV-Strahlungsfeld aus mindestens einfach, aber auch an den Seitenflächen des Reaktorraumes mehrfach reflektieren UV-Strahlung erzeugt wird, sodass der UV-Sensor das in der Reaktorkammer herrschende quasi gleichmäßig diffuse Strahlungsfeld wesentlich genauer detektieren kann und beispielsweise etwaige Veränderungen der UV-Transmission mit einer außerordentlichen Genauigkeit erkannt werden können, wobei der UV-Sensor bevorzugterweise auf einen räumlich bestimmten Sensorbereich des homogenes UV-Strahlungsfeldes ausgerichtet ist, wodurch über die Auswerteeinheit das detektierte Sensorsignal des Sensorbereichs über das gesamte homogene UV-Strahlungsfeld extrapolierbar ist und das Ergebnis der Extrapolation aufgrund der Homogenität des UV-Strahlungsfeldes ein höchstgenaues Abbild des tatsächlich erzeugten Desinfektionsergebnisses wiederspiegelt.

Nachfolgend ist die Vorrichtung zur Überwachung einer UV-Desinfektionsanlage anhand von Zeichnungen näher beschrieben. Es zeigen:
- Fig. 1: eine Draufsicht auf die UV-Desinfektionsanlage und
- Fig. 2: eine geschnittene Seitenansicht der UV-Desinfektionsanlage gemäß Fig. 1.

Die UV-Desinfektionsanlage besteht im Wesentlichen aus einem wasser- oder flüssigkeitsdurchfluteten Reaktorkammer 1, die von einem Quarzfenster 7 abgedeckt ist, durch den UV-LEDs 2 ihr UV-Licht in die Reaktorkammer 1 einstrahlen, wobei das UV-Licht auf der als Reflektionsfläche 4 ausgebildeten und dem Quarzfenster 7 gegenüber liegenden Fläche reflektiert wird, sodass ein benachbart der UV-LEDs 2 angeordneter UV-Sensor 3 dieses nach doppelten Durchlauf durch die Reaktorkammer 1 detektieren kann, wobei die detektierten Werte an eine zeichnerisch nicht dargestellten Auswerteeinheit weitergegeben und von dieser aufbereitet und ausgewertet werden.

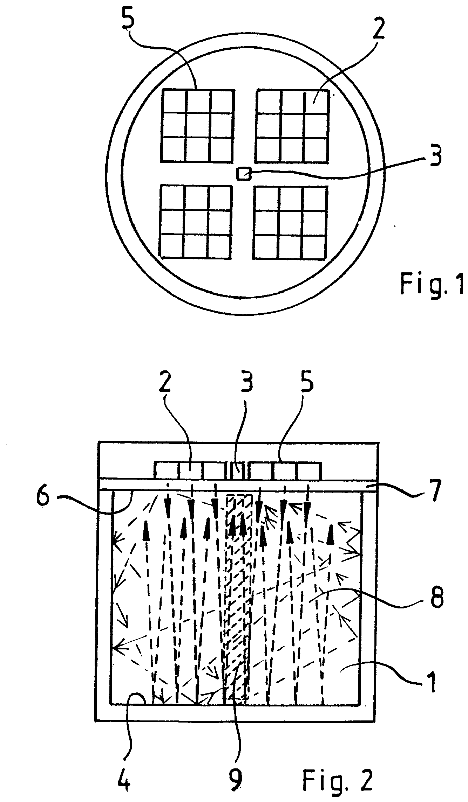

In der Reaktorkammer 1 wird ein homogenes UV-Strahlungsfeld 8 erzeugt, wobei darunter nicht notwendigerweise, wie in Fig. 2 nur grob skizziert, eine annähernde parallele Hin- und Rückstrahlung des UV-Lichtes angenommen werden muss, da in der Reaktorkammer 1 aufgrund der einfachen oder mehrfachen Reflektion des UV-Lichts auch an den seitlichen Reflektionsflächen der Reaktorkammer 1 darin ein stark diffuse Strahlung vorherrschen kann, die trotzdem weitestgehend homogen ist, sodass der von dem UV-Sensor 3 erfasste Sensorbereich 9 bzw. dessen detektierte Werte über das Volumen der gesamten Reaktorkammer 1 extrapoliert werden können um exakte Werte für das gesamte Volumen zu generieren, ebenso wie über die Auswerteeinheit Veränderungen der UV-Transmission durch im Wasser gelöste oder partikuläre Substanzen oder etwa eine Belagsbildung auf der wasserzugewandten Seite des Quarzfensters 7 detektiert werden können.

## Patentansprüche

1. Vorrichtung zur Überwachung einer UV-Desinfektionsanlage mit einer wasserdurchfluteten Reaktorkammer (1) und mindestens einer darin einstrahlen könnend angeordneten UV-Strahlungsquelle sowie mit einem UV-Sensor (3) und eine Auswerteeinheit für das Sensorsignal, **dadurch gekennzeichnet, dass** die Strahlungsquelle aus UV-LEDs besteht, die mindestens auf einer Seite der Reaktorkammer (1) angeordnet sind und dass der UV-Sensor (3) auf der gleichen Seite zwischen den UV-LEDs (2) angeordnet ist und dass mindestens die dieser Seite gegenüberliegende Innenfläche der Reaktorkammer (1) als Reflektionsfläche (4) für das UV-Licht ausgebildet ist.

2. Vorrichtung nach Anspruch 1, **dadurch gekennzeichnet, dass** die UV-LEDs (2) auf einem Trägermodul (5) angeordnet sind und dass ihre Anzahl der erforderlichen Reinigungs- und Desinfektionsleistung angepasst ist.

3. Vorrichtung nach einem der vorgenannten Ansprüche, **dadurch gekennzeichnet, dass** sich das Trägermodul (5) außerhalb der durchfluteten Reaktorkammer (1) befindet und die UV-LEDs (2) unmittelbar auf einem Quarzfenster (7) aufliegen, dessen den LEDs (2) abgewandte Seite eine Reaktorinnenwand (6) und den oberen Abschluss der Reaktorkammer (1) bildet.

4. Vorrichtung nach einem der vorgenannten Ansprüche, **dadurch gekennzeichnet, dass** die UV-LEDs (2) so platziert und ausgerichtet sind, dass in die Reaktorkammer (1) ein gleichmäßiges homogenes UV-Strahlungsfeld (8) einstrahlbar ist.

5. Vorrichtung nach einem der vorgenannten Ansprüche, **dadurch gekennzeichnet, dass** der UV-Sensor (3) auf einem räumlich bestimmten Sensorbereich (9) des homogenen UV-Strahlungsfeldes (8) ausgerichtet ist.

6. Vorrichtung nach Anspruch 5, **dadurch gekennzeichnet, dass** über die Auswerteeinheit das detektierte Sensorsignal des Sensorbereichs (9) über das gesamte homogene UV-Strahlungsfeld (8) extrapolierbar ist.

7. Arbeitsverfahren zur Überwachung einer UV-Desinfektionsanlage mittels einer Vorrichtung gemäß einem der vorgenannten Ansprüche, **dadurch gekennzeichnet, dass** die UV-LEDs (2) in der Reaktorkammer (1) ein gleichmäßiges homogenes UV-Strahlungsfeld (8) erzeugen und dass der UV-Sensor (3) das von den UV-LEDs (2) in die Reaktorkammer (1) eingestrahlte UV-Licht nach mindestens zweimaliger Durchquerung der Reaktorkammer (1) in einem räumlich begrenzten Sensorbereich (9) erfasst.

8. Arbeitsverfahrens nach Anspruch 1, **dadurch gekennzeichnet, dass** in der Auswerteeinheit das Sensorsignals des UV-Sensors (3), ausgehend von den in dem Sensorbereich (9) detektierten Werten, diese über das gesamte homogene UV-Strahlungsfeld (8) extrapoliert werden.

9. Arbeitsverfahren nach einem der vorgenannten Ansprüche 7 oder 8, **dadurch gekennzeichnet, dass** die Auswerteeinheit etwaige Veränderungen der UV-Transmission detektiert.

10. Arbeitsverfahren nach Anspruch 9, **dadurch gekennzeichnet, dass** die Auswerteeinheit eine Belagsbildung auf der dem Wasser zugewandten Seite des Quarzfensters (7) und/ oder eine Schwächung der UV-Transmission durch im Wasser gelöste oder partikuläre Substanzen detektiert.
